# EUROPÄISCHE PATENTSCHRIFT

(11) **EP 0 784 505 B1**
(45) Veröffentlichungstag und Bekanntmachung des Hinweises auf die Patenterteilung: **26.08.1998**
(21) Anmeldenummer: 95935373.1
(22) Anmeldetag: 25.09.1995
(51) Int. Cl.: B01J 8/22, B01J 19/00

(54) **SCHLAMMPHASENREAKTOR UND DESSEN VERWENDUNG**
SLUDGE PHASE REACTOR AND ITS USE
REACTEUR A PHASE BOUEUSE ET SON UTILISATION

(30) Priorität: 07.10.1994 DE 4435839
(43) Veröffentlichungstag der Anmeldung: 23.07.1997
(73) Patentinhaber: BAYER AG, 51368 Leverkusen (DE)
(72) Erfinder: WITT, Harro, D-25712 Kuden (DE); ZARNACK, Uwe, Jens, D-25541 Brunsbüttel (DE); BECKHAUS, Heiko, D-51381 Leverkusen (DE)
(86) Internationale Anmeldenummer: EP9503786
(87) Internationale Veröffentlichungsnummer: WO9611052

(56) Entgegenhaltungen:
- DE-A- 1 792 246
- DE-B- 1 135 663
- DE-B- 1 245 598
- FR-A- 2 018 420
- GB-A- 1 413 565
- US-A- 2 334 553
- US-A- 3 400 051
- CHIMIE ET INDUSTRIE, Bd. 106, Nr. 7, März 1973 Seiten 483-493, J. ROUSSEL 'L'UTILISATION DE L'AZOTE LIQUIDE POUR LE CONTROLE THERMIQUE DES REACTIONS CHIMIQUES'
- PATENT ABSTRACTS OF JAPAN vol. 016 no. 168 (C-0932) ,22.April 1992 & JP,A,04 016229 (HITACHI LTD) 21.Januar 1992,

## Beschreibung

Schlammphasenreaktionen sind Reaktionen, an denen mindestens eine feinteilige feste und eine flüssige Phase beteiligt sind.

Stark exotherme Schlammphasenreaktionen verlangen eine effektive Ableitung der Reaktionswärme. Schlammphasenreaktoren weisen daher aufwendige Wärmeaustauscher-Konstruktionen im Inneren des Reaktionsbehälters auf, siehe z. B. US-A 3 243 268 oder EP-A 263 935. Bei den bekannten Schlammphasenreaktoren erfolgt der Wärmeaustausch dadurch, daß die Reaktionsmasse an sogenannten "Field"-Wärmeaustauscherrohren oder an einseitig geschlossenen Siederohren, in denen das Wärmeübertragungsmittel und der entstehende Dampf im Gegenstrom geführt werden, umgepumpt wird. Nachteilig dabei ist die - bezogen auf den Reaktorraum - unterschiedliche Strömungsgeschwindigkeit der Reaktionsmasse und der dadurch bedingte schlechte Wirkungsgrad des Wärmetauschers. Die unterschiedliche Strömungsgeschwindigkeit der Reaktionsmasse bewirkt strömungsberuhigte Zonen, in denen sich die an der Reaktion beteiligte Feststoffkomponente absetzen kann.

Erfindungsgemäß wird nun vorgeschlagen, als Wärmetauscher einen von dem Wärmeübertragungsmittel im wesentlichen nur in einer Richtung durchströmten Behälter vorzusehen, wobei der Behälter von einer Vielzahl von Durchtrittskanälen mit rundem Querschnitt für die Reaktionsmasse ausgestattet ist. Erfindungsgemäß wird im Wärmeaustauscher demgemäß die Reaktionsmasse "invers" im Vergleich zu herkömmlichen Verdampfungswärmeaustauschern geführt.

Aus der DE-B 11 35 663 ist ein derartiger Wärmeaustauscher zur Durchführung von Polymerisationsreaktionen von flüssigen Medien bei Temperaturen unterhalb -80°C bekannt.

Gegenstand der vorliegenden Erfindung ist ein Schlammphasenreaktor für exotherme Schlammphasenreaktionen unter Beteiligung einer Gasphase, der folgende Elemente enthält:
a) einen Reaktionsbehälter;
b) einen Wärmeaustauscher in Form eines von dem Kühlmittel und der Reaktionsmasse durchströmten Ringraumes innerhalb des Reaktionsbehälters, wobei der Ringraum von der Reaktionsmasse unter- und überdeckt ist,
c) wobei der Ringraum eine Vielzahl vertikaler Durchtrittskanäle mit rundem Querschnitt für die Reaktionsmasse aufweist und das Wärmeaustauschmittel den Ringraum zwischen den Durchtrittskanälen für die Reaktionsmasse durchströmt;
d) einen zentralen freien Strömungsraum innerhalb des Ringraumes für die Rückströmung der Reaktionsmasse;
e) einen zentralen Rührer, der die Reaktionsmasse zwischen dem zentralen freien Strömungsraum und dem Ringraum im Umlauf fördert,
f) wobei der Rührer als Begasungsrührer ausgebildet ist, der aus dem Gasraum oberhalb der Reaktionsmasse Gas ansaugt, und
g) der Rührer die Reaktionsmasse in den zentralen Strömungsraum nach unten fördert und eine Strömung in den vertikalen Durchtrittskanälen nach oben bewirkt.

Vorzugsweise wird der Wärmetauscher-Ringraum von Reaktionsmasse und Wärmeübertragungsmittel im wesentlichen gleichsinnig durchströmt. Insbesondere werden die Druchtrittskanäle für die Reaktionsmasse durch den Ringraum vertikal nach oben durchströmt. In diesem Falle wird bevorzugt der Rührer am unteren Austritt des zentralen freien Strömungsraumes angeordnet und mindestens eine der Reaktionskomponenten in unmittelbarer Nähe des Rührers zugeführt, so daß einerseits eine schnelle Verteilung der Reaktionskomponente in der Reaktionsmasse erfolgt und andererseits die zugeführte Reaktionskomponente mit der Reaktionsmasse sehr schnell in den Wärmeaustauscher eingeleitet wird.

Zur kontinuierlichen Durchführung der Reaktion ist oberhalb des Flüssigkeitsspiegels im Reaktionsbehälter ein Überlauf vorgesehen, aus dem reagierte Reaktionsmasse kontinuierlich abziehbar ist.

Als Wärmeübertragungsmittel wird vorzugsweise ein bei Reaktionstemperatur verdampfendes Mittel eingesetzt, das in flüssiger Form am Boden des Wärmeaustauscher-Ringraumes zugeführt wird und am Kopf des WärmeaustauscherRingraumes in gasförmiger Form abgezogen wird. Besonders bevorzugt wird als Wärmeübertragungsmittel Wasser eingesetzt und der Wärmeaustausch unter Erzeugung von Dampf durchgeführt. Die Kühlmitteltemperatur wird vorzugsweise über eine Druckhaltung des erzeugten Dampfes eingestellt.

Zur Einführung der Gasphase in den Reaktor ist der Rührer als Begasungsrührer ausgebildet bzw. weist ein zusätzliches Begasungselement auf.

Der erfindungsgemäße Schlammphasenreaktor mit Begasungsrührer ist insbesondere für die Durchführung von Schlammphasenhydrierungen von aromatischen Nitroverbindungen, besonders bevorzugt für die Hydrierung von Dinitrotoluolen unter Erzeugung der entsprechenden Diamine, geeignet.

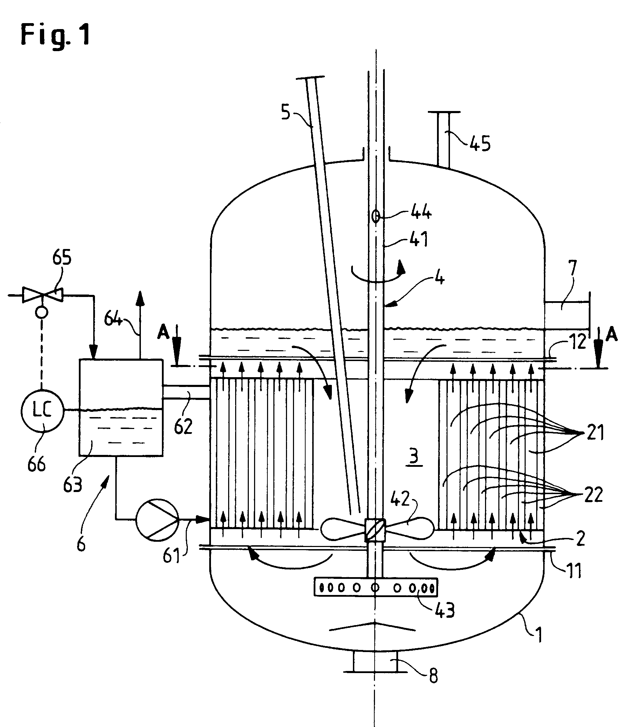

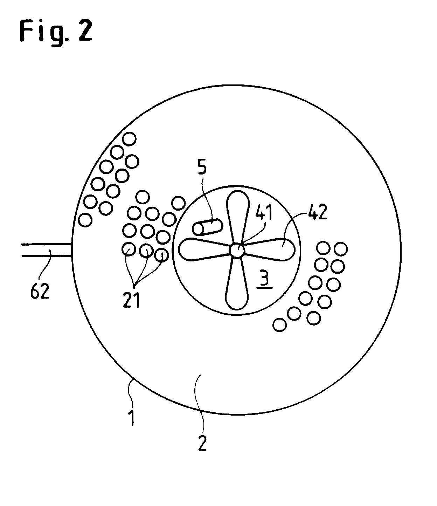

Die Erfindung wird anhand der beigefügten Fig. 1 und 2 näher erläutert:
- Fig. 1: zeigt einen Querschnitt durch den erfindungsgemäßen Schlammphasenreaktor.
- Fig. 2: zeigt einen Querschnitt A-A durch den Reaktor gemäß Fig. 1.

Der Reaktor gemäß Fig. 1 besteht aus einem Reaktionsbehälter 1, der aus drei Teilen aufgebaut ist, die mittels Flanschen 11 und 12 zusammengeflanscht sind. Der mittlere Teil des Reaktionsbehälters 1 enthält den ringförmigen Wärmeaustauscher 2, der aus einem allseits geschlossenen Ringraum besteht und von einer Vielzahl von Durchgangsrohren 21, den die Reaktionsmasse aufsteigend entlang den angedeuteten Pfeilen durchströmt, durchtreten wird. In dem verbleibenden Zwischenraum 22 des Ringraums 2 befindet sich das Wärmeaustauschmittel. Die Rückströmung der Reaktionsmasse erfolgt, wie durch Pfeile angedeutet, durch den zentralen freien Strömungsraum 3 innerhalb des ringförmigen Wärmeaustauschers 2. Die umlaufende Strömung der Reaktionsmasse wird durch den Rührer 4 mit zentraler, außerhalb des Reaktors angetriebener Rührerachse 41 und Rührblatt 42 bewirkt. Dabei ist das Rührblatt 42 in Höhe des Austritts aus dem zentralen Strömungsraum 3 nach unten angeordnet. In der dargestellten Ausführungsform ist die Rührerachse 41 als Hohlachse ausgebildet und weist unterhalb des Rührblattes 42 eine Begasungseinrichtung 43 auf. Das Gas für die Begasung der Reaktionsmasse wird aus dem Gasraum im oberen Teil des Reaktionsbehälters 1 mittels Ansaugöffnungen 44 in der Rührerachse 41 angesaugt. Über den Gaszufuhrstutzen 45 wird ein vorgegebener Gasdruck gewährleistet. Bei dem bevorzugten Einsatz des erfindungsgemäßen Reaktors für die Hydrierung aromatischer Nitroverbindungen wird Wasserstoff bei einem Druck von 10 bis 40 bar eingesetzt. Die Frischwasserstoffzufuhr kann vorteilhafter direkt in die Reaktionsmasse erfolgen. Die aromatische Nitroverbindung wird über Zufuhrleitung 5 in unmittelbarer Nähe des Rührblattes 42 eingeleitet. Als feinteilige Feststoffphase ist in der Reaktionsmasse der Hydrierungskatalysator, z.B. Edelmetalle oder Nickel auf Trägerteilchen wie Kohle, SiO₂, Al₂O₃ usw. bzw. Raney-Nickel-Katalysatoren in der Reaktionsmasse dispergiert. Das Produkt wird kontinuierlich am Überlauf 7 abgezogen, so daß ein gleichbleibender Flüssigkeitsspiegel erhalten bleibt. Der Wärmeaustauscher-Ringraum wird ferner aus einem Wärmeaustauschmittel-Kreislauf 6 gespeist. Das Wärmeaustauschmittel, vorzugsweise Wasser knapp unterhalb des Siedepunktes wird bei 61 in den Wärmeaustauscher unten eingeleitet. Wasserdampf tritt bei 62 oben aus dem Wärmeaustauscher aus und wird dem Dampfabscheider 63 zugeleitet. Pfeil 64 deutet die Ableitung des Dampfes zur Energierückgewinnung an. Das Zuflußventil für Kühlwasser 65 wird mittels einer Niveauregelung 66 für den Wasserstand im Dampfabscheider 63 gesteuert. Beim bevorzugten Einsatz des erfindungsgemäßen Reaktors für die Hydrierung aromatischer Nitroverbindungen wird der Kühlmitteldruck 64 so geregelt, daß im Reaktor eine Temperatur von 120 bis 250°C aufrechterhalten wird.

Die Ziffern in Fig. 2 bezeichnen die gleichen Elemente wie in Fig. 1. Die Durchtrittsrohre 21 sind nur teilweise dargestellt. Der Wärmeaustauscher 2 kann 100 bis 3000 Durchtrittsrohre 21 aufweisen, je nach Nennweite der Rohre (25-10 mm) und Durchmesser des Wärmeaustauschers (bis zu 3 m).

## Patentansprüche

1. Schlammphasenreaktor für exotherme Schlammphasen-Reaktionen unter Beteiligung einer Gasphase, enthaltend
a) einen Reaktionsbehälter;
b) einen Wärmeaustauscher in Form eines von dem Wärmeaustauschmittel und der Reaktionsmasse durchströmten Ringraumes, der von der Reaktionsmasse unter- und überdeckt ist,
c) wobei der Ringraum eine Vielzahl vertikaler Durchtrittskanäle mit rundem Querschnitt für die Reaktionsmasse aufweist und das Wärmeaustauschmittel den Ringraum zwischen den Durchtrittskanälen für die Reaktionsmasse durchströmt;
d) einen zentralen freien Strömungsraum für die Rückströmung der Reaktionsmasse innerhalb des Ringraumes;
e) einen zentralen Rührer, der die Reaktionsmasse zwischen dem zentralen freien Strömungsraum und dem Ringraum im Umlauf fördert,
f) wobei der Rührer als Begasungsrührer ausgebildet ist, der aus dem Gasraum oberhalb der Reaktionsmasse Gas ansaugt, und
g) der Rührer die Reaktionsmasse in den zentralen Strömungsraum nach unten fördert und eine Strömung in den vertikalen Durchtrittskanälen nach oben bewirkt.

2. Schlammphasenreaktor nach Anspruch 1, dadurch gekennzeichnet, daß der Rührer am Austritt des zentralen freien Strömungsraumes angeordnet ist und wandgängig ausgebildet ist.

3. Schlammphasenreaktor nach Anspruch 1 oder 2, dadurch gekennzeichnet, daß mindestens ein Reaktionspartner in unmittelbarer Nähe des Rührers in die Reaktionsmasse eingeleitet wird.

4. Verwendung des Reaktors nach einem der Ansprüche 1 bis 3 zur Durchführung von Schlammphasen-Hydrierungen.

5. Verwendung nach Anspruch 4 zur Hydrierung von aromatischen Nitroverbindungen.

6. Verwendung nach Anspruch 5 zur Hydrierung von Dinitrotoluolen.

7. Verfahren zur Durchführung von Schlammphasen-Hydrierungen unter Einsatz eines Reaktors nach einem der Ansprüche 1 bis 3, dadurch gekennzeichnet, daß als Kühlmittel Wasser eingesetzt wird und nutzbarer Dampf erzeugt wird.

## Claims

1. A sludge phase reactor for exothermic sludge phase reactions involving a gas phase, comprising
a) a reaction vessel;
b) a heat exchanger in the form of an annular chamber through which the heat exchange medium and the reaction mass flow and which is covered above and below by the reaction mass,
c) wherein the annular chamber comprises a plurality of vertical ducts of round cross section for passage of the reaction mass, and the heat exchange medium flows through the annular chamber between the reaction mass passage ducts;
d) a free central flow chamber for the return flow of the reaction mass inside the annular chamber;
e) a central agitator, which circulates the reaction mass between the free central flow chamber and the annular chamber,
f) wherein the agitator takes the form of a sparging agitator, which draws in gas from the gas chamber above the reaction mass, and
g) the agitator conveys the reaction mass downwards into the central flow chamber and produces an upward flow in the vertical passage ducts.

2. A sludge phase reactor according to claim 1, characterised in that the agitator is arranged at the outlet from the free central flow chamber and is constructed so as to be able to pass through the wall thereof.

3. A sludge phase reactor according to claim 1 or claim 2, characterised in that at least one reacting agent is introduced into the reaction mass in the immediate vicinity of the agitator.

4. Use of the reactor according to any one of claims 1 to 3 for performing sludge phase hydrogenation.

5. Use according to claim 4 for hydrogenating aromatic nitro compounds.

6. Use according to claim 5 for hydrogenating dinitrotoluenes.

7. A method of performing sludge phase hydrogenation using a reactor according to any one of claims 1 to 3, characterised in that water is used as a coolant and useful steam is generated.

## Revendications

1. Réacteur à phase boueuse pour réactions à phase boueuse exothermiques avec participation d'une phase gazeuse, comprenant
a) une cuve de réaction ;
b) un échangeur de chaleur ayant la forme d'un espace annulaire traversé par l'agent de refroidissement et la masse en réaction à l'intérieur de la cuve de réaction, l'espace annulaire étant recouvert par le haut et par le bas de la masse en réaction,
c) l'espace annulaire présentant un grand nombre de canaux verticaux de passage de section ronde pour la masse en réaction et l'agent de refroidissement circulant dans l'espace annulaire entre les canaux de passage pour la masse en réaction ;
d) un espace libre central de circulation à l'intérieur de l'espace annulaire pour la circulation de retour de la masse en réaction ;
e) un agitateur central, qui assure la circulation de la masse en réaction entre l'espace libre central de circulation et l'espace annulaire,
f) l'agitateur étant construit comme agitateur d'absorption de gaz, qui aspire le gaz de l'espace gazeux au-dessus de la masse en réaction, et
g) l'agitateur transportant la masse en réaction vers le bas dans l'espace libre central de circulation et provoquant une circulation vers le haut dans les canaux de passage verticaux.

2. Réacteur à phase boueuse suivant la revendication 1, caractérisé en ce que l'agitateur est disposé à la sortie de l'espace libre central de circulation et construit de façon à repousser vers la paroi.

3. Réacteur à phase boueuse suivant la revendication 1 ou 2, caractérisé en ce qu'au moins un composant de réaction est introduit dans la masse en réaction à proximité immédiate de l'agitateur.

4. Utilisation du réacteur suivant l'une des revendications 1 à 3 pour la réalisation d'hydrogénation de phases boueuses.

5. Utilisation suivant la revendication 4 pour l'hydrogénation de nitrocomposés aromatiques.

6. Utilisation suivant la revendication 5 pour l'hydrogénation de dinitrotoluols.

7. Procédé de réalisation d'hydrogénation de phases boueuses avec utilisation d'un réacteur suivant l'une des revendications 1 à 3, caractérisé en ce que de l'eau est utilisée comme agent de refroidissement et de la vapeur utilisable est produite.
